# EUROPÄISCHE PATENTANMELDUNG

(11) **EP 3 847 954 A1**
(43) Veröffentlichungstag der Anmeldung: **14.07.2021**
(21) Anmeldenummer: 20151457.7
(22) Anmeldetag: 13.01.2020
(51) Int. Cl.: A61B 5/01, G16H 50/20, A61B 3/14

(54) **VORRICHTUNG ZUR BESTIMMUNG DER RELATIVEN AUGENTEMPERATUR**

(71) Anmelder: Friedrich-Alexander-Universität Erlangen-Nürnberg, 91054 Erlangen (DE)
(72) Erfinder: HERRMANN, Martin, 91077 Neunkirchen a. Brand (DE); MARDIN, Christian, 91056 Erlangen (DE); HOHBERGER, Bettina, 91245 Simmelsdorf (DE); LÄMMER, Robert, 91054 Erlangen (DE); BERGUA, Antonio, 91054 Erlangen (DE); KREBS, Johann Ferdinand, 91052 Erlangen (DE); HOSARI, Sami, 91054 Erlangen (DE)
(74) Vertreter: Dr. Gassner & Partner mbB

(57) **Zusammenfassung**

Die Erfindung betrifft eine Vorrichtung zur Bestimmung der relativen Augentemperatur, umfassend
eine Augentemperaturmesseinrichtung (1) zur kontaktlosen Messung der Augentemperatur zumindest eines Auges und zur Übermittlung zumindest eines gemessenen Augentemperaturwerts an eine Datenverarbeitungseinrichtung (2),
eine Körpertemperaturmesseinrichtung (3) zur Messung der Körpertemperatur und zur Übermittlung zumindest eines gemessenen Körpertemperaturwerten an die Datenverarbeitungseinrichtung (2), und
eine Datenverarbeitungseinrichtung (2) zur Erfassung und Verarbeitung des zumindest einen Augentemperaturwerts und des zumindest einen Körpertemperaturwerts, wobei die Datenverarbeitungseinrichtung so hergerichtet ist, dass ein Quotient Q und/oder eine Differenz des zumindest einen Augentemperaturwerts und des zumindest einen Körpertemperaturwerts gebildet und angezeigt wird.

## Beschreibung

Die Erfindung betrifft eine Vorrichtung zur Bestimmung der relativen Augentemperatur.

Nach dem Stand der Technik ist es beispielsweise aus der US 5,297,554 bekannt, die Augentemperatur mittels Kontaktlinsen zu messen, an denen eine Temperaturmesseinrichtung angebracht ist. Das Applizieren derartiger Kontaktlinsen ist umständlich. Abgesehen davon kann mit der bekannten Vorrichtung lediglich die absolute Augentemperatur gemessen werden. Zudem besteht ein Verletzungs- und Infektionsrisiko während des Einsetzens und Tragens der Kontaktlinse.

Die US 7,665,848 B2 offenbart eine Vorrichtung, bei der mittels Sensoren eine Messung des Drucks, der Bewegung, der Temperatur und der Feuchtigkeit der Augen gemessen und die Messwerte nachfolgend mittels eines Computerprogramms verarbeitet werden. Damit kann ein Maß eines Arbeitsstresses ermittelt werden.

Die JP 2005-192762 A beschreibt eine Messeinrichtung zur Erfassung der Verdampfung von Tränenflüssigkeit. Die Vorrichtung umfasst einen Sensor zur Messung der Temperatur und der Feuchtigkeit der Umgebungsluft.

Aufgabe der Erfindung ist es, anhand der Messung der Augentemperatur eine Aussage über das Entstehen, Bestehen und Fortschreiten von Krankheiten und/oder Degenerationen zu treffen. Zu diesem Zweck soll eine geeignete Vorrichtung angegeben werden.

Diese Aufgabe wird durch die Merkmale des Patentanspruchs 1 gelöst. Zweckmäßige Ausgestaltungen ergeben sich aus den Merkmalen der abhängigen Patentansprüche.

Nach Maßgabe der Erfindung wird eine Vorrichtung zur Bestimmung der relativen Augentemperatur vorgeschlagen, umfassend
eine Augentemperaturmesseinrichtung zur kontaktlosen Messung der Augentemperatur zumindest eines Auges und zur Übermittlung zumindest eines gemessenen Augentemperaturwerts an eine Datenverarbeitungseinrichtung,
eine Körpertemperaturmesseinrichtung zur Messung der Körpertemperatur und zur Übermittlung zumindest eines gemessenen Körpertemperaturwerts an die Datenverarbeitungseinrichtung, und
eine Datenverarbeitungseinrichtung zur Erfassung und Verarbeitung des zumindest einen Augentemperaturwerts und des zumindest einen Körpertemperaturwerts, wobei die Datenverarbeitungseinrichtung so hergerichtet ist, dass ein Quotient und/oder eine Differenz des zumindest einen Augentemperaturwerts und des zumindest einen Körpertemperaturwerts gebildet und angezeigt wird.

Die vorgeschlagene Vorrichtung ermöglicht erstmals eine automatisierte Bestimmung der "relativen Augentemperatur". Unter dem Begriff "relative Augentemperatur" wird eine Augentemperatur verstanden, welche bezüglich der Körpertemperatur des Patienten korrigiert bzw. normiert ist. Zu diesem Zweck kann beispielsweise ein Quotient aus gemessenen Augen- und Körpertemperaturwerten gebildet werden. Es ist aber auch denkbar, die "relative Augentemperatur" beispielsweise durch Differenzbildung anzugeben.

Es hat sich überraschenderweise gezeigt, dass anhand der relativen Augentemperatur Erkrankungen und Degenerationen diagnostiziert werden können. Es können insbesondere neuronale, okulare und systemische Erkrankungen und Degenerationen erkannt werden. Z. B. können Gefäßverschlüsse, arterielle Stenosen, Glaukome und viele andere Krankheiten diagnostiziert werden.

Erfindungsgemäß wird die Augentemperatur kontaktlos gemessen. Eine kontaktlose Messung ist beispielsweise mittels Infrarotabstrahlung, Laserreflektion oder dgl. möglich. Die vorgeschlagene kontaktlose Messung der Augentemperatur ist schnell, einfach und genau. Ein mechanischer Kontakt mit dem Auge und ein damit verbundenes Verletzungsrisiko werden vermieden.

Insbesondere aus dem Quotienten aus den Augen- und Körpertemperaturwerten lassen sich schnell und einfach Erkrankungen und Degenerationen erkennen. Die vorgeschlagene Vorrichtung eignet sich insbesondere zum Screenen und routinemäßigen Untersuchen einer großen Anzahl von Patienten. Die Untersuchung kann schnell, einfach und kostengünstig durchgeführt werden.

Vorteilhafterweise ist die Augentemperaturmesseinrichtung so hergerichtet, dass damit die Augentemperatur ausschließlich im Bereich der Pupille gemessen wird. Eine Messung der Augentemperatur im Bereich der Pupille ist besonders aussagekräftig. Indem für die Augentemperaturmessung der Bereich der Pupille festgelegt wird, lassen sich gemessene Augentemperaturwerte unterschiedlicher Patienten miteinander vergleichen. - Es ist auch möglich, die Temperatur des Auges zu messen, indem beispielsweise die Temperatur des Auges oder des Augenanhangsgebildes gemessen wird. Im Bereich der Pupille kann die Fläche der Pupille, ein Flächenabschnitt der Pupille und/oder ein Punkt im Bereich der Pupille, insbesondere der Mittelpunkt, der Pupille gemessen werden.

Nach einer vorteilhaften Ausgestaltung ist die Augentemperaturmesseinrichtung eine Thermografieeinrichtung. Mit der Thermografieeinrichtung lässt sich schnell und einfach ein Bild herstellen, welches die Oberflächentemperatur der Augen wiedergibt. Die Thermografieeinrichtung umfasst zweckmäßigerweise zwei IR-Sensoren. Die IR-Sensoren können an einem, vorzugsweise brillenartigen, Gestell angebracht sein. Das Gestell ist zweckmäßigerweise so ausgestaltet, dass ein Abstand zwischen den IR-Sensoren und/oder eine Stütze zum Abstützen des Gestells auf der Nasenwurzel einstellbar ist. Damit können die IR-Sensoren in geeigneter Weise gegenüber den zu vermessenden Augen angeordnet werden. - Auch die Körpertemperaturmesseinrichtung kann am Gestell angebracht sein. Die vorgeschlagene Vorrichtung ist mobil. Sie lässt sich leicht handhaben. Das Gestell kann Bestandteil eines stationären oder mobilen Geräts sein.

Die Augentemperaturmesseinrichtung und/oder die Körpertemperaturmesseinrichtung können eine Vorrichtung zur drahtlosen Datenübertragung umfassen. Das vereinfacht weiter die Handhabung der Augentemperaturmesseinrichtung und/oder der Körpertemperaturmesseinrichtung.

Es ist aber auch denkbar, die Augentemperaturmesseinrichtung stationär auszubilden. Dazu kann die Vorrichtung ähnlich einer Spaltlampe ausgestaltet oder mit einer Spaltlampe kombiniert werden. Die Vorrichtung kann auch in ein bestehendes Gerät eingebaut werden, z. B. in eine Ganzfeld-Kuppel Einrichtung.

Nach einer besonders vorteilhaften Ausgestaltung ist die Datenverarbeitungseinrichtung zur Bilderkennung hergerichtet, so dass eine Fläche der Pupille erkannt und ausschließlich die von dieser Fläche abgestrahlte Wärme erfasst wird. Mittels der Bilderkennung können insbesondere Bilder analysiert werden, welche von der Thermografieeinrichtung geliefert werden.

Nach einer weiteren Ausgestaltung ist die Datenverarbeitungseinrichtung so ausgestaltet, dass für zumindest ein Auge aus mehreren Augentemperaturmesswerten ein Mittelwert und/oder ein Median und/oder eine Standardabweichung und/oder eine Varianz gebildet wird. D. h. es können z. B. im Bereich der Pupille eines jeden Auges mehrere Augentemperaturwerte gemessen und daraus ein Mittelwert oder dgl. gebildet werden.

Die Datenverarbeitungseinrichtung kann eine Datenbank umfassen, in welcher vorgegebene Quotienten- und/oder Differenzbereiche bestimmten Krankheiten zugeordnet sind. In diesem Fall ist die Datenverarbeitungseinrichtung vorteilhafterweise so ausgestaltet, dass die Quotienten und/oder Differenzen mit den in der Datenbank hinterlegten Quotienten- und oder Differenzbereichen verglichen und bei Übereinstimmung die zum jeweiligen Quotienten- und/oder Differenzbereich hinterlegte Krankheit angezeigt wird. Das ermöglicht schnell und einfach eine Diagnose einer Vielzahl von Erkrankungen und/oder Degenerationen.

Unter Verwendung der vorgeschlagenen Vorrichtung lässt sich ein Diagnostizierverfahren mit folgenden Schritten durchführen:
Bereitstellen der erfindungsgemäßen Vorrichtung in einem Raum mit einer vorgegebenen Umgebungstemperatur,
Akklimatisieren eines Patienten in dem Raum für eine vorgegebene Zeit,
Messung der Augentemperatur und Übermittlung zumindest eines gemessenen Augentemperaturwerts an die Datenverarbeitungseinrichtung,
Messung der Körpertemperatur und Übermittlung zumindest eines gemessenen Körpertemperaturwerts an die Datenverarbeitungseinrichtung, und
Bildung eines Quotienten und/oder einer Differenz aus den Augen- und Körpertemperaturwerten mittels der Datenverarbeitungseinrichtung und Anzeige des Quotienten und/oder der Differenz.

Das vorgeschlagene Diagnostizierverfahren ermöglicht eine Erkennung insbesondere neurologischer, ophthalmologischer, vaskulärer und systemischer Erkrankungen. Als Beispiele können genannt werden: Multiple Sklerose, Morbus Alzheimer, Gefäßverschlüsse, Glaukome, arterielle Stenosen, Zustände nach Operationen sowie Diabetes.

Nachfolgend wird ein Ausführungsbeispiel der Erfindung anhand der Zeichnungen näher erläutert. Es zeigen:
- Fig. 1: ein Blockschaltbild der Vorrichtung,
- Fig. 2: eine Augentemperaturmesseinrichtung,
- Fig. 3: ein Thermografiebild, und
- Fig. 4: die Temperaturverteilung entlang der Linie L1 in Fig. 3.

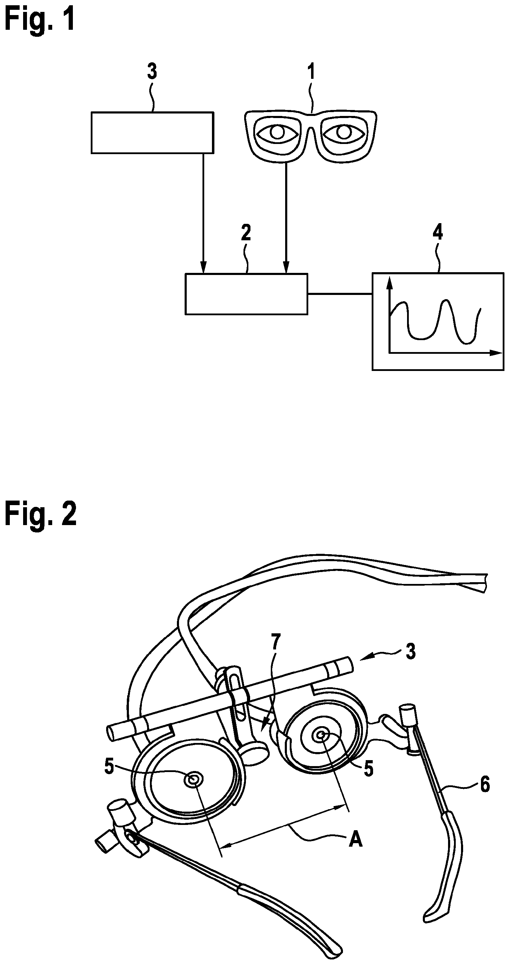

Fig. 1 zeigt ein Blockschaltbild der erfindungsgemäßen Vorrichtung. Eine schematisch gezeigte Augentemperaturmesseinrichtung 1 zur kontaktlosen Messung der Augentemperatur beider Augen ist zur Übermittlung von gemessenen Augentemperaturwerten mit einer Datenverarbeitungseinrichtung 2 verbunden. Die Verbindung zur Übermittlung von Messwerten kann drahtgebunden oder drahtlos sein. Zur drahtlosen Verbindung eignet sich beispielsweise eine Bluetooth-Verbindung oder dgl.

Mit dem Bezugszeichen 3 ist eine Körpertemperaturmesseinrichtung bezeichnet. Dabei handelt es sich zweckmäßigerweise um ein Ohrthermometer, welches mit einem IR-Sensor versehen ist. Daneben eignet sich insbesondere auch ein Stirn- oder Schläfenthermometer, welche ebenfalls mit einem IR-Sensor versehen sind. Die von einem Ohrthermometer oder einem Stirn- oder Schläfenthermometer gelieferten Messwerte liegen üblicherweise unterhalb der tatsächlichen Körpertemperatur. Die Messwerte können mittels eines voreingestellten Korrekturwerts korrigiert werden.

Auch die Körpertemperaturmesseinrichtung 3 kann entweder drahtgebunden oder drahtlos zur Übermittlung der Messwerte mit der Datenverarbeitungseinrichtung 2 verbunden sein. Mit dem Bezugszeichen 4 ist eine Anzeigeeinrichtung bezeichnet, beispielsweise ein Bildschirm, auf welcher die ermittelten Messwerte, Quotienten, Thermografiebilder oder dgl. angezeigt werden.

Fig. 2 zeigt eine Augentemperaturmesseinrichtung 3. Es handelt sich dabei um eine Thermografieeinrichtung, bei welcher zwei IR-Sensoren 5 in einem brillenartigen Gestell 6 angebracht sind. Das Gestell 6 ist so ausgestaltet, dass ein Abstand A zwischen den IR-Sensoren 5 einstellbar ist. Ferner kann eine Stütze 7 zum Abstützen des Gestells 6 auf einer Nasenwurzen (hier nicht gezeigt) verschwenkt werden. Damit kann ein weiterer Abstand zwischen den IR-Sensoren 5 und den Augen des Patienten eingestellt werden.

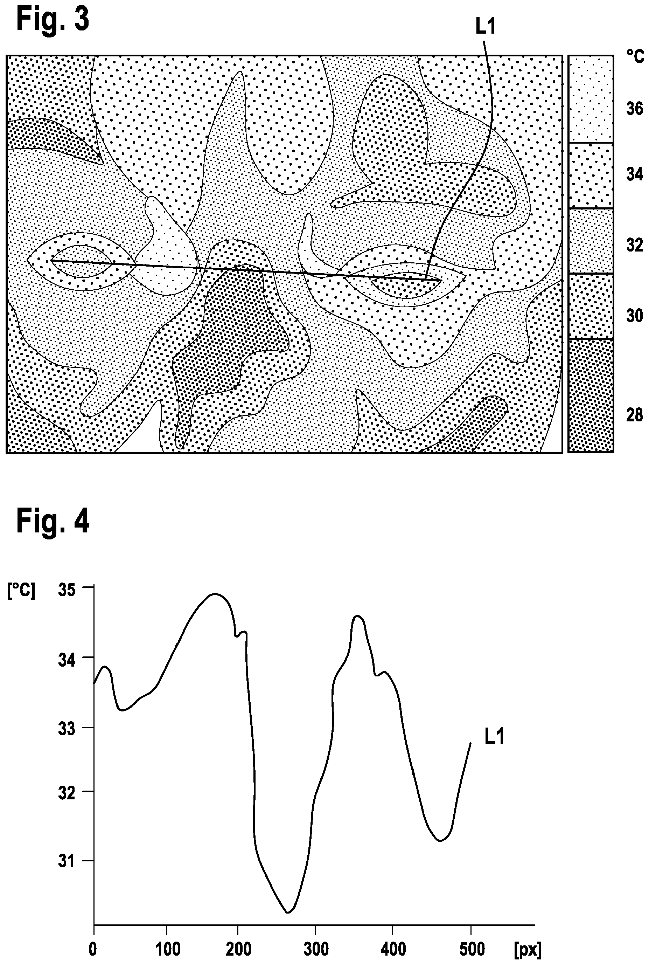

Fig. 3 zeigt ein Thermografiebild, welches mit einer Thermografieeinrichtung aufgenommen worden ist. Erkennbar ist, dass sich im Zentrum des linken Auges des Patienten ein dunkler Fleck befindet. Der untersuchte Patient leidet hier unter glaukomatöser Optikusatrophie. Fig. 4 zeigt das dazugehörige Temperaturprofil entlang der in Fig. 3 gezeigten Linie L1. Daraus ist erkennbar, dass das linke Auge im Vergleich zum rechten Auge eine geringere Augentemperatur aufweist.

Zur einfachen Diagnose von Erkrankungen, Degenerationen, Augenveränderungen und bei Laserbehandlung des Auges werden die insbesondere im Bereich der Pupille gemessenen Augentemperaturen ins Verhältnis zur gemessenen Körpertemperatur gesetzt. Insbesondere können die Augentemperaturen im Bereich der Fläche der Pupille und/oder im Mittelpunkt der Pupille gemessen werden.

Die Messung der Augentemperatur kann punktuell im Bereich der Pupille erfolgen. Zweckmäßigerweise erfolgt die Messung der Augentemperatur unter Verwendung einer Bilderkennung. Dabei wird in einem Thermografiebild zunächst der Bereich der Pupille erkannt. Sodann wird die von diesem Bereich abgestrahlte Wärme zu einem Mittelwert der Augentemperatur verarbeitet.

Bei gesunden Patienten ergeben sich für den dabei ermittelten Quotienten Q Werte im Bereich von 0,9464 bis 0,9896 (0,968 ± 0,0216). Wenn ein Patient z. B. an einem Glaukom erkrankt ist, ergeben sich für den Quotienten Q Werte im Bereich von 0,8724 bis 0,9256. Die Quotienten Q eines kranken Patienten unterscheiden sich also deutlich vom Quotienten Q eines gesunden Patienten. Infolgedessen eignet sich die Vorrichtung zur schnellen und einfachen Diagnose von Erkrankungen und/oder Degenerationen.

### Bezugszeichenliste

- 1: Augentemperaturmesseinrichtung
- 2: Datenverarbeitungseinrichtung
- 3: Körpertemperaturmesseinrichtung
- 4: Anzeigeeinrichtung
- 5: IR-Sensor
- 6: Gestell
- 7: Stütze

- A: Abstand

## Patentansprüche

1. Vorrichtung zur Bestimmung der relativen Augentemperatur, umfassend
eine Augentemperaturmesseinrichtung (1) zur kontaktlosen Messung der Augentemperatur zumindest eines Auges und zur Übermittlung zumindest eines gemessenen Augentemperaturwerts an eine Datenverarbeitungseinrichtung (2),
eine Körpertemperaturmesseinrichtung (3) zur Messung der Körpertemperatur und zur Übermittlung zumindest eines gemessenen Körpertemperaturwerts an die Datenverarbeitungseinrichtung (2), und
eine Datenverarbeitungseinrichtung (2) zur Erfassung und Verarbeitung des zumindest einen Augentemperaturwerts und des zumindest einen Körpertemperaturwerts, wobei die Datenverarbeitungseinrichtung so hergerichtet ist, dass ein Quotient Q und/oder eine Differenz des zumindest einen Augentemperaturwerts und des zumindest einen Körpertemperaturwerts gebildet und angezeigt wird.

2. Vorrichtung nach Anspruch 1, wobei die Augentemperaturmesseinrichtung (1) so hergerichtet ist, dass damit die Augentemperatur ausschließlich im Bereich der Pupille gemessen wird.

3. Vorrichtung nach einem der vorhergehenden Ansprüche, wobei die Augentemperaturmesseinrichtung (1) eine Thermografieeinrichtung ist.

4. Vorrichtung nach einem der vorhergehenden Ansprüche, wobei die Thermografieeinrichtung zwei IR-Sensoren (5) umfasst.

5. Vorrichtung nach einem der vorhergehenden Ansprüche, wobei die IR-Sensoren (5) in einem Gestell (6), vorzugsweise einem brillenartigen Gestell, angebracht sind.

6. Vorrichtung nach einem der vorhergehenden Ansprüche, wobei das Gestell (6) Bestandteil eines stationären oder mobilen Geräts ist.

7. Vorrichtung nach einem der vorhergehenden Ansprüche, wobei das Gestell (6) so ausgestaltet ist, dass ein Abstand zwischen den IR-Sensoren (5) und/oder eine Stütze (7) zum Abstützen auf der Nasenwurzel einstellbar ist.

8. Vorrichtung nach einem der vorhergehenden Ansprüche, wobei am Gestell (6) die Körpertemperaturmesseinrichtung (3) zur Messung der Körpertemperaturwerte angebracht ist.

9. Vorrichtung nach einem der vorhergehenden Ansprüche, wobei die Augentemperaturmesseinrichtung (1) und/oder die Körpertemperaturmesseinrichtung (3) eine Vorrichtung zur drahtlosen Datenübertragung umfassen.

10. Vorrichtung nach einem der vorhergehenden Ansprüche, wobei die Datenverarbeitungseinrichtung (2) zur Bilderkennung hergerichtet ist, so dass eine Fläche der Pupille erkannt und ausschließlich die von dieser Fläche abgestrahlte Wärme erfasst bestimmt wird.

11. Vorrichtung nach einem der vorhergehenden Ansprüche, wobei die Datenverarbeitungseinrichtung (2) so ausgestaltet ist, dass für jedes Auge aus mehreren Augentemperaturmesswerten ein Mittelwert und/oder ein Median und/oder eine Standardabweichung und/oder eine Varianz gebildet wird.

12. Vorrichtung nach einem der vorhergehenden Ansprüche, wobei die Datenverarbeitungseinrichtung (2) eine Datenbank umfasst, in welcher vorgegebene Quotienten- und/oder Differenzbereiche bestimmten Krankheiten zugeordnet sind.

13. Vorrichtung nach einem der vorhergehenden Ansprüche, wobei die Datenverarbeitungseinrichtung (2) so ausgestaltet ist, dass die Quotienten Q mit den in der Datenbank hinterlegten Quotientenbereichen verglichen und bei Übereinstimmung die zum jeweiligen Quotientenbereich hinterlegte Krankheit angezeigt wird.
